(19) Europäisches Patentamt
European Patent Office
Office européen des brevets

(11) Publication number: 0 207 642 A2

(12) # EUROPEAN PATENT APPLICATION

(21) Application number: 86304250.3

(22) Date of filing: 04.06.86

(51) Int. Cl.⁴: C11D 1/83 , A61K 7/08

(30) Priority: 21.06.85 GB 8515762

(43) Date of publication of application:
07.01.87 Bulletin 87/02

(84) Designated Contracting States:
AT BE CH DE FR GB IT LI NL SE

(71) Applicant: DEUTSCHE ICI GMBH
Lyonerstrasse 36 Postfach 710330
D-6000 Frankfurt/Main - Niederrad 71(DE)

(72) Inventor: Dahms, Gird
Auf Den Poethen 52
D-5620 Velbert 15(DE)

(74) Representative: Colens, Alain M.G.M. et al
Imperial Chemical Industries PLC Legal
Department: Patents P.O. Box 6 Bessemer
Road
Welwyn Garden City Hertfordshire AL7
1HD(GB)

(54) **Composition based on anionic and nonionic surfactants, their preparation and uses thereof.**

(57) The invention related to surfactant formulations which are mixtures of sulphonate or isethionate anionic surfactants and a defined class of nonionic surfactants. These formulations retain the valuable properties of the specified anionic surfactants, but also give high water solubility and a low viscosity.

EP 0 207 642 A2

This invention relates to surfactant compositions, based on anionic and nonionic surfactants. They find application in personal care compositions such as shampoos, shower gels and liquid soaps.

Certain anionic surfactants, namely sulphonate esters and isethionates have valuable surfactant properties and are biodegradable, but they have limited solubility in water and are viscous pastes, as normally prepared. In the case of certain sulphonate esters, this solubility can be as low as 0.5 to 2.0% by weight in water at 25°C. Solubility makes formulation difficult and it is not usually possible to make clear compositions which are favoured by the consumer. The compositions also have much lower viscosity than the sulphonate ester on its own.

We have now found that by admixture with certain nonionic surfactants it is possible to prepare compositions which retain the valuable properties of the specified anionic surfactants, but also give high water solubility and a low viscosity.

Our invention provides a surfactant composition comprising a mixture of

A) an anionic surfactant which is selected from, or is a mixture of :

1. a sulphonate surfactant of the formula :

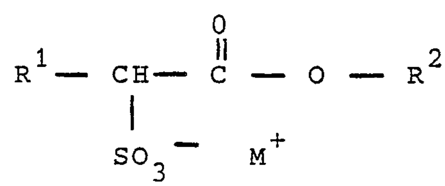

and

2. an isethionate surfactant of the formula :

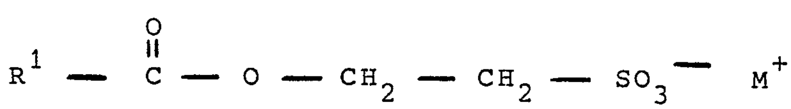

where $R^1$ is a $C_{12}$ -$C_{22}$ hydrocarbon group;

$R^2$ is a $C_1$ -$C_5$ hydrocarbon group;

M is an alkali metal ion or ammonium or quaternary ammonium.

$R^1$ and $R^2$ may contain substituents.

and

B) a nonionic surfactant selected from nonionic surfactants such that composition A and B in a 1:2 weight ratio without added water has a viscosity of less than 10.000 mPas when measured with a Brookfield RVT viscometer at 25 °C.

The hydrocarbon group $R^1$ may be any well known group as used in surfactant technology. It may be a synthetically prepared grouping or may be derived from natural sources, plant or animal, by known techniques. Tallow or vegetable oils are convenient, and there may be at least partially hydrogenated if it is desired to reduce unsaturation. Examples are hydrogenated tallow, lauryl, palm and coco-derived groups.

The hydrocarbon group $R^2$ is preferably methyl or ethyl.

The alkali metal is preferably sodium or potassium.

The non-ionic surfactant has to be selected by small scale trial using the criteria defined above. Suitable materials will be found in the classes of It is also desirable that an aqueous solution, say 10% mixed surfactants in water, is transparent and free from haziness and suspended solids.

Examples

A series of nonionic surfactants were tested using the above procedure.

Sodium methyl $\alpha$ -sulphotallowate was used as the anionic surfactant. A sample containing 66% water was a paste of viscosity greater than 3.300.000 mPas. This surfactant was blended with the selected nonionic surfactant and the water content was reduced to below 2% by evaporation.

Viscosity of the product mixture was measured at 25°C. The following results were obtained.

| Composition N° | non-ionic surfactant composition | Viscosity |
|---|---|---|
| 1 | POE(6) $C_{13}/C_{15}$ alcohol (Renex 706) | 780 |
| 2 | POE(9) nonylphenol (Renex 698) | 6.730 |
| 3 | POE(11)oleic acid (Atlas G5511) | 8.660 |
| 4 | POE(20)sorbitanmonolaurate (Tween 20) | 4.000 |
| 5 | POE(9) sorbitanmonolaurate (Atlas EL496) | 1.350 |
| 6 | POE(25) Castorwax (Atlas G 1292) | >33.000 |
| 7 | POP(15)-POE(20) - 12 - Hydroxy stearylalcohol | >16.000 |

ethoxylated and/or propoxylated hydroxyl and amine containing compounds. Propoxyl groups may be present and it may be advantageous to vary the content of ethylene and propylene oxides and their random or block relationship in order to optimise the properties of the composition.

Ethoxylated acids alcohols and polyols have been found to provide effective nonionic surfactants. Examples of groups of such surfactants are $C_9$ -$C_{22}$ alcohols polyethers, $C_9$ -$C_{22}$ fatty acid ester polyethers, alkyl phenol polyethers hexital polyethers, in all of which the polyether group contains from 2 to 25 ethylene oxide-derived groups. Alcohol and acid derivatives are preferred because their good biodegradability does not detract from the inherent biodegradability of certain preferred anionic surfactants. The weight ratio of the two surfactants A and B is in the range 10:90 to 90:10 Preferably it is in the range 40:60 to 60:40.

The composition is prepared by mixing the surfactants A and B, preferably under high shear mixing conditions at temperatures conveniently in the range 20°C to 60°C.

The composition may be incorporated in a wide range of surfactant formulations, and are especially useful in personal care products such as shampoos, shower gels, liquid soaps and similar skin cleaning products. For these purposes, alkyl sulpho tallowate methyl esters in admixture with ethoxylated fatty acides and alcohols have been found to give good results.

Compositions of the invention may be formulated with perfumes, colouring agents, emollients, other surfactants, oils, and other cosmetics ingredients. The inclusion of some amine oxide surfactant gives stable foams in aqueous solution.

The invention is further illustrated by the following test procedure to determine whether a given non-ionic surfactant is suitable or not for use in the invention and the subsequent Examples.

Test Procedure

A nonionic surfactant, to be suitable for use in the present invention should be selected which is liquid at temperatures up to 25°C. A mixture is then prepared with the selected anionic surfactant, for example, the sodium salt of methyl α -sulphotallowate. The anionic surfactant will normally be in the form of an aqueous paste or dispersion. Sufficient non-ionic surfactant is mixed to give a weight ratio of one part anionic to two parts nonionic surfactant, calculated on the basis of water-free components. Since there will probably be water present in at least the anionic surfactant, this is then removed by evaporation under vacuum to a low level, below 2%. Mixing is carried out under high shear conditions. The sample is then evaluated. In particular, the viscosity is measured, and satisfactory non-ionic surfactants for the purpose of this invention will give a viscosity (as measured by a Brookfield RVT viscometer) of less than 10.000 mPas.

'Renex', ' Tween' and 'Atlas' are trademarks of ICI Americas Inc.

It will be seen that Composition Nos 1 -5 give good results according to the test criteria.

Composition Nos 6 and 7 fail the test and do not therefore form part of the present invention.

Samples of Composition Nos 1 -5 were diluted to 10% weight solutions in water.

The following observations were made on these solutions.

| Solution of Composition NO. | Viscosity of Solution mPas | Viscosity of Solution with 2% $CaCl_2$ | Tur- bidity * |
|---|---|---|---|
| 1 | 33 | 330 | 64 |
| 2 | 16 | 36 | 81 |
| 3 | 14 | 15 | 129 |
| 4 | 13 | 20 | 108 |
| 5 | 15 | 10906 | 74 |

* The measurement was made with a DroH TRM-L meter, calibrated in Formazin units.

It will be noted that Compositions 1 and 5 are especially interesting in that they have lowest viscosities when undiluted, the lowest turbidity when diluted and show high viscosity when 2% calcium chloride is added, thus allowing the preparation of high viscosity and gelled products.

Formulation I

A formulation was prepared as follows

| | |
|---|---|
| Composition No 1 | 10 parts |
| Amine Oxide (Synprolam' DMO ex ICI) | 10 parts |
| Water | 4 parts |
| Calcium Chloride | 1 part |

The formulation gave a foam of viscosity 94 000 mPas which was stable for over three months.

This formulation is useful in the preparation of shaving foam and for fire extinguishing.

Formulation II

A series of formulations for bath and shower gel was prepared as follows

| | |
|---|---|
| Composition No 1 | 5-15 parts |
| $C_{13-15}$ alcohol ether sulphate (70% solution in water) | |
| "Synperonic" 3570, ex ICI | 15-30 parts |
| Amine oxide ("Synprolam"DMO) | 3-5 parts |
| Perfume | q.s. |
| Water | to 100 parts |

The products give satisfactory cleaning and a good re-fatted skin feel.

Formulation III

A series of formulations for shampoos were prepared as follows

| | |
|---|---|
| Composition No 1 | 10-20 parts |
| $C_{13-15}$ alcohol ether sulphate | |
| ("Synperonic"3570) | 20-30 parts |
| Amine oxide ("Synprolam" DMO) | 3-7 parts |
| Protein hydrolysate | 3-5 parts |
| Perfume | q.s. |
| Water | to 100 parts |

The products are highly biodegradable, gave good washing results, yet is very mild in use.

Formulation IV

Liquid soap formulations were prepared as follows

| | |
|---|---|
| Composition Nó 1 | 20-40 parts |
| $C_{13-15}$ alchohol ether sulphate | |
| ("Synperonic" 3570) | 0-15 parts |
| Amine oxide ("Synprolam" DMO) | 0-5 parts |
| POP(15)Stearyl alcohol ("Arkamol" E) | 0-4 parts |
| Perfume | q.s. |
| Water | to 100 parts |

In all composition ranges, a biodegradable mild liquid soap was obtained with good refatted skin feel after use.

**Claims**

1. A surfactant composition comprising a mixture of

A) an anionic surfactant which is selected from or is a mixture of

1. a sulphonate surfactant of the formula:

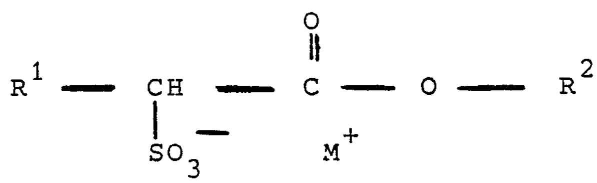

and

2. an isethionate surfactant of the formula

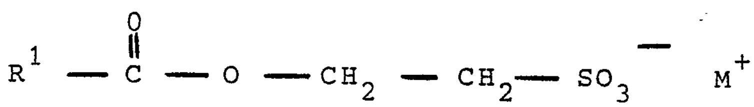

where $R^1$ is a $C_{12}$ -$C_{22}$ hydrocarbon group;

$R^2$ is a $C_1$ -$C_5$ hydrocarbon group;

M is an alkali metal ion or ammonium or quaternary ammonium;

$R^1$ and $R^2$ may contain substituents;

and

B) an nonionic surfactant selected from nonionic surfactants such that composition A and B in a 1:2 weight ratio without added water has a viscosity of less than 10,000 mPas when measured with a Brookfield RVT Viscometer at 25° C.

2. A surfactant composition according to claim 1 wherein $R_1$ is a hydrogenated tallow, lauryl, palm and coco-derived group and $R_2$ is ethyl or methyl.

3. A surfactant composition according to claim 1 wherein $M^+$ is a sodium or potassium cation.

4. A surfactant composition according to claim 1 wherein the anionic surfactant is methyl alpha-sulphotallowate.

5. A surfactant composition according to claim 1 wherein the nonionic surfactant is a ethoxylated and/or propoxylated hydroxyl or amine containing compounds.

6. A surfactant composition according to claim 5 wherein the nonionic surfactant is a $C_9$-$C_{22}$ alcohol polyether, $C_9$-$C_{22}$ fatty acid ester polyether, alkyl phenol polyether, hexital polyether with from 2 to 25 ethylene oxide-derived groups.

7. A surfactant composition according to claim 1, 5 and 6 wherein the nonionic surfactant is an ethoxylated sorbitanmonolaurate.

8. An aqueous formulation comprising a composition according to claims 1 to 7 and which further contains an alcohol ether sulphate, amine oxide, perfume, protein hydrolysate or stearyl alcohols.

9. Use of a composition or formulation according to claims 1 to 8 in shampoos, shower gels and liquid soaps.

CLAIMS FOR CONTRACTING STATE: AT

1. A method for the preparation of a surfactant composition which comprises mixing

A) an anionic surfactant which is selected from or is a mixture of

1. a sulphonate surfactant of the formula :

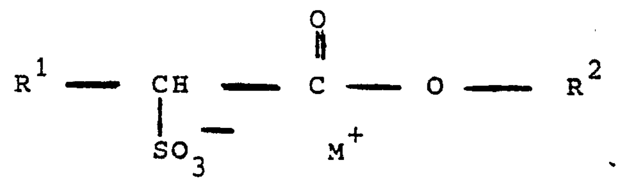

and

2. an isethionate surfactant of the formula

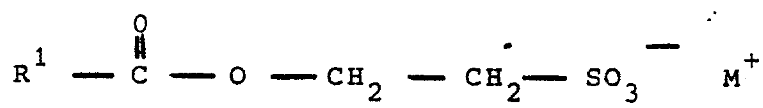

where R' is a $C_{12}$ -$C_{22}$ hydrocarbon group;

R² is a $C_1$ -$C_5$ hydrocarbon group;

M is an alkali metal ion or ammonium or quaternary ammonium;

R' and R² may contain substituents;

and

B) an nonionic surfactant selected from nonionic surfactants such that composition A and B in a 1:2 weight ratio without added water has a viscosity of less than 10,000 mPas when measured with a Brookfield RVT Viscometer at 25° C.

2. **A method according to claim 1** wherein $R_1$ is a hydrogenated tallow, lauryl, palm and coco-derived group and $R_2$ is ethyl or methyl.

3. **A method according to claim 1** wherein $M^+$ is a sodium or potassium cation.

4. A surfactant composition according to claim 1 wherein the anionic surfactant is methyl alpha-sulphotallowate.

5. **A method according to claim 1** wherein the nonionic surfactant is a ethoxylated and/or propoxylated hydroxyl or amine containing compounds.

6. **A method according to claim 5** wherein the nonionic surfactant is a $C_9$-$C_{22}$ alcohol polyether, $C_9$-$C_{22}$ fatty acid ester polyether, alkyl phenol polyether, hexital polyether with from 2 to 25 ethylene oxide-derived groups.

7. **A method according to claim 1** 5 and 6 wherein the nonionic surfactant is an ethoxylated sorbitanmonolaurate.

8. A method according to claim 1 to 7 wherein the surfactants are part of an aqueous formulation which further contains an alcohol ether sulphate, amine oxide, perfume, protein hydrolysate or stearyl alcohol.

9. Use of a composition or formulation according to claims 1 to 8 in shampoos, shower gels and liquid soaps.